(19) Europäisches Patentamt

European Patent Office

Office européen des brevets

(11) **EP 1 481 738 A2**

(12) **EUROPEAN PATENT APPLICATION**

(43) Date of publication:
**01.12.2004 Bulletin 2004/49**

(51) Int Cl.7: **B06B 1/06**

(21) Application number: **04016458.4**

(22) Date of filing: **26.03.1999**

(84) Designated Contracting States:
**AT BE CH CY DE DK ES FI FR GB GR IE IT LI LU MC NL PT SE**

(30) Priority: **26.03.1998 US 79428 P**

(62) Document number(s) of the earlier application(s) in accordance with Art. 76 EPC:
**99914182.3 / 1 066 119**

(71) Applicants:
• **Exogen Inc.**
**Piscataway, NJ 08855 (US)**
• **RUTGERS, THE STATE UNIVERSITY OF NEW JERSEY**
**Piscataway, NJ 08854-8010 (US)**

(72) Inventors:
• **Cornejo, Ivan A.**
**Highland Park NJ 08904 (US)**

• **Jadidian, Bahram**
**H-10 Dayton NJ 08810 (US)**
• **Safari, A.**
**Princeton Junction NJ 08550 (US)**
• **Winder, Alan A.**
**Westport CT 06880 (US)**

(74) Representative: **Laufhütte, Dieter, Dr.-Ing. et al**
**Lorenz-Seidler-Gossel**
**Widenmayerstrasse 23**
**80538 München (DE)**

Remarks:
This application was filed on 13 - 07 - 2004 as a divisional application to the application mentioned under INID code 62.

(54) **ULTRASONIC ARRAYS OF FLEXIBLE PIEZOELECTRIC TRANSDUCER ELEMENTS**

(57) A piezoelectric composite transducer element comprising:

a fiber sheet formed from fibers extruded from a composition containing a piezoelectric material, the fiber sheet being rolled about a common axis to define a generally spiral configuration, overlapping segments of the spiral defining a gap therebetween, the gap being filled with a first polymeric material.

FIGURE 2A

EP 1 481 738 A2

**Description**

BACKGROUND OF THE INVENTION

1. Technical Field

Novel flexible piezoelectric transducer elements and large-area flexible arrays of such transducers are described herein. Also described are methods for manufacture of the flexible transducer elements and transducer arrays. Additionally, use of the transducer elements or transducer arrays in medical therapeutic ultrasound applications, e.g., for promoting the healing of bone fractures and tissue wounds, medical diagnostic ultrasound applications and nondestructive testing are also described herein.

2. Background of Related Art

The use of ultrasound to therapeutically treat and evaluate bone fractures is known. Impinging ultrasonic pulses having appropriate dosage parameters, e.g., frequency, acoustic intensity, duty cycle, pulse repetition rate and total treatment time, and administered externally at the fracture site has been determined to accelerate the natural healing of, for example, bone fractures. For patients with reduced healing capacity, such as elderly persons with osteoporosis, ultrasonic therapy may promote healing of bone injuries that would otherwise require prosthetic replacement or leave the patient permanently disabled.

U.S. Patent No. 4,530,360 to Duarte describes a basic non-invasive therapeutic technique and apparatus for applying ultrasonic pulses externally on the skin of the patient at a location adjacent to the bone fracture site. The applicator described in the '360 patent has a plastic tube which serves as a grip for the operator, an RF plug attached to the plastic tube for connection to an RF source, and internal cabling connected to a rigid ultrasonic transducer. To apply the ultrasound pulses during treatment, an operator manually holds the applicator in place until the treatment is complete. The '360 patent also describes a range of RF signals for creating the ultrasound, ultrasound power density levels, a range of duration for each ultrasonic pulse, and a range of ultrasonic pulse frequencies. U.S. Patent Nos. 5,003,695; 5,211,160 and 5,762,616 relate to ultrasonic body treatment systems that include rigid transducer elements and structure for fixing the transducer adjacent body tissue.

While these prior art systems provide accelerated healing of soft tissue wounds and bone fractures, the transducer elements and the arrays of such elements are too rigid to conform to large area contours of the body such as, for example, the contours of the hip and spine. It would be very desirable to provide flexible transducer elements and flexible, unitary arrays containing such flexible transducer elements that conform to body contours to facilitate the effective application of therapeutic ultrasonic dosages.

Previous attempts have been made to provide flexible transducer elements. For example, U.S. Patent No. 4,227,111 discloses a flexible, low density, piezoelectric composite transducer in which the piezoelectric composite is formed by cojoining a polymer phase and a piezoelectric phase in such a manner that each individual phase is interconnected in all three orthogonal dimensions. As another example, U.S. Patent No. 4,233,477 discloses a flexible, composite, acoustical energy transducer made from ferroelectric, piezoelectric and/or electrostrictive materials arranged in the same x-y plane within a polymer having electroelastic properties. The composite is flexible so it can be made into different shapes such as flat, bent, and cylindrical and/or secured to a curved or differently shaped body. Yet another example is U.S. Patent No. 5,691,960 which discloses acoustic transducer panels fabricated as a composite of a piezoelectric or electrostrictive ceramic material and a polymeric material.

A common deficiency of flexible transducer elements is a lack of physical integrity. Due to poor adhesion between ceramic and polymeric components, physical separation of the components may occur upon bending of the transducer element. Physical separation of the components can introduce air pockets that interfere with operation of the transducer element. Maintenance of physical integrity is especially troublesome where the transducer element is intended for therapeutic applications and includes a matching layer or where a monolithic array of such transducer elements is flexed.

It would be desirable to provide a flexible piezoelectric ceramic/polymer composite transducer element or a flexible, unitary transducer array of such flexible transducer elements that can be conformed to the contours of the human anatomy in need of therapeutic stimulation.

SUMMARY OF THE INVENTION

Novel flexible piezoelectric composite transducer elements and large-area flexible arrays of such transducer elements for use in therapeutic applications have been discovered. The novel transducer arrays include at least two flexible composite transducer elements arranged in adjacent relation with polymer-filled spaces therebetween, electrode surfaces applied to opposite surfaces of the array and a matching layer applied to, and substantially coextensive with, one of the electrode surfaces.

In one embodiment, composite transducer elements used to make the arrays are obtained by dicing and filling a piezoelectric wafer. That is, the composite transducer elements are obtained by forming a first and second set of channels in the piezoelectric material, forming a film of polymeric material on the piezoelectric material and covering the film with a second polymeric material in an amount sufficient to fill the first and second set of channels.

In another embodiment, composite transducer elements used to make the arrays are obtained by rolling a sheet formed from extruded fibers of a piezoelectric material into a spiral configuration, and fining the gaps in the spiral with a polymeric material.

Methods for making large-area flexible piezoelectric composite transducer arrays have also been discovered. In the method, a plurality of the flexible composite transducer elements are arranged in adjacent relation to define spaces therebetween to form an array, the spaces are filled with a polymeric material, electrodes are applied to opposite surfaces of the array and a matching layer is applied to one of the electrode surfaces.

A method for using the piezoelectric composite transducer elements and the flexible transducer arrays in therapeutic applications has also been discovered. In the method, flexible transducer elements or flexible transducer arrays of the transducer elements can be applied to a section of the human anatomy in need of therapeutic stimulation and that section of the human anatomy is then exposed to a dosage amount of acoustic energy.

The transducer elements and large-area flexible transducer arrays described herein possess sufficient flexibility such that the transducers advantageously permit acoustic energy generated by the transducer to be efficiently applied and coupled to the contours of the human anatomy for therapeutic and diagnostic applications.

BRIEF DESCRIPTION OF THE DRAWINGS

Preferred embodiments of the invention are described below with reference to the drawing, which are described as follows:

FIG. 1A is a schematic view of a piezoelectric material having interconnecting channels formed therein to be used in forming one type of composite transducer element in accordance with the present invention;

FIG. 1B is a cross-sectional schematic view of the material of FIG. 1A with a matching layer formed thereon;

FIG. 1C is a cross-sectional schematic view of the material of FIG. 1B with an electrode surface applied to opposite surfaces of the material and a matching layer formed on one surface thereof to form a composite transducer element;

FIG. 2A is a schematic perspective view of a fiber sheet rolled into a spiral configuration;

FIG. 2B is a schematic top view of a fiber sheet rolled into a spiral configuration;

FIG. 3A is a schematic top view of an array of spiral composite transducer elements used to form a flexible transducer array in accordance with the present invention;

FIG. 3B is a cross-sectional schematic view of the array shown in FIG. 3A;

FIG. 3C is a cross-sectional schematic view of the array shown in FIG. 3B with an electrode surface applied to opposite surfaces of the array and a matching layer applied to one of the electrode surfaces; and,

FIG. 4 is a cross-sectional schematic view of an array of the composite transducer elements of FIG. 1C used to form a flexible transducer array in accordance with the present invention.

DESCRIPTION OF THE PREFERRIED EMDODIMENTS

A flexible piezoelectric composite transducer array in accordance with this disclosure includes an array containing a plurality of piezoelectric composite transducer elements arranged in adjacent relation to define spaces therebetween with a polymeric material disposed in the spaces, electrodes applied to opposite surfaces of the array and a matching layer applied to, and substantially coextensive with, one of the electrode surfaces. The composite transducer elements which make up the transducer array are themselves flexible, e.g., have a flexibility of at least 2.0 mm as measured by a four-point bend test. The novel transducer arrays made from these flexible transducer elements are likewise flexible and can conform to different contours of the human anatomy when employed for therapeutic application.

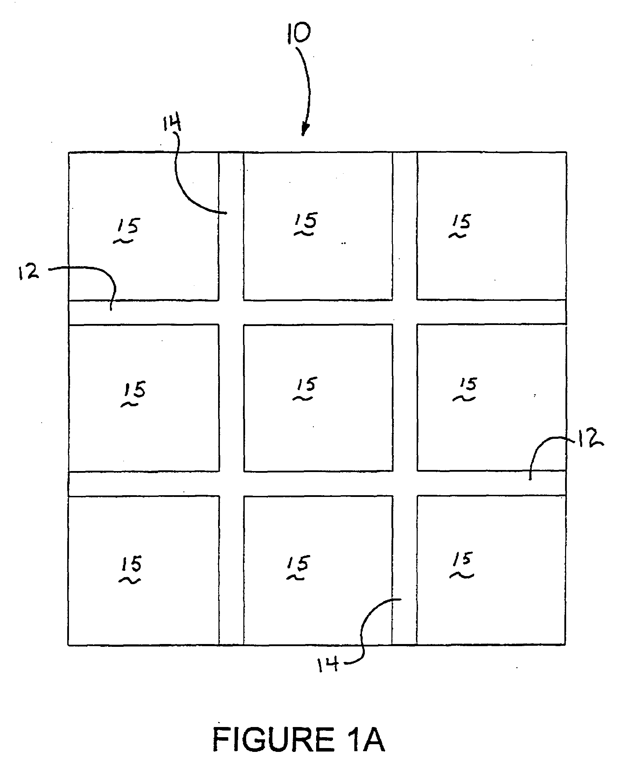

As seen in the embodiments shown in FIG. 1A-1C, one type of composite transducer element for use herein can be formed by first providing a piezoelectric material 10. Suitable piezoelectric materials for this composite include PZT powders commercially available from Morgan Matroc, Inc., ceramic, single-crystal relaxor ferroelectric, lead zirconate titanate $Pb(Zr,Ti)O_3$, lead metaniobate $Pb(Nb_2O_6)$, modified lead titanate $PbTi_3$ such as $(Pb,Ca)TiO_3$ and $(Pb,Sm)TiO_3$, barium titanate $BaTiO_3$, PMN-PT$(1-x)Pb(Mg_{1/3}Nb_{2/3})O_3$-$xPbTiO_3$, PZN-PT/BT $Pb(Zn_{1/3}Nb_{2/3})O_3$-$xPbTiO_3$-$BaTiO_3$, $(1-x)Pb(Zn_{1/3}, Nb_{2/3})O_3$-$x(yPbTiO_3$-$(1-y)PbZrO_3)$ and the like. In general, the piezoelectric material 10 is processed by first pressing the material uniaxially at a pressure from about 15 ksi to about 50 ksi and then sintering the pressure material at a temperature ranging from about 1000°C to about 1400°C and preferably from about 1150°C to about 1300°C for a time period not exceeding about 2 hours. A lead source, e.g., a mixture of PbO and $ZrO_2$, can be added to piezoelectric material 10 when sintering to compensate for any loss of lead from the material 10.

Following processing, piezoelectric material 10 is subjected to dicing. Initially, a first set of channels 12 are formed parallel to each other and within material 10 (see FIG. 1A). Techniques for forming channels 12 are within the purview of one skilled in the art. Typically, the width of each channel 12 will range from about 25 to about 1,200 microns and preferably from about 200 to about 600 microns. Preferably, when forming the channel in parallel juxtaposition, the distance between the channels will range from about 200 to about 400 microns.

A second set of channels 14 is then be formed parallel to one another and angularly oriented with respect to each channel 12. In general, the sets of interconnecting channels can intersect at an angle of between about 5° and about 90° and preferably between about 30° and about 90°. It should be understood that an angle other than 90° between the chancels may impart preferential bending in a desired plane. Therefore, perpendicular sets of channels are preferred. Thus, as generally depicted in FIG. 1A, each of channels 12 and 14 are linear and intersect to define islands 15 of piezoelectric material. Techniques for forming channels 14 are within the purview of one skilled in the art. Typically, the width of each channel 14 can be the same or different as each of channel 12. Thus, the width of channel 14 will ordinarily range from about 25 to about 1,200 microns and preferably from about 200 to about 600 microns. Preferably, when forming the channels in parallel juxtaposition, the distance between the channels will range from about 200 to about 400 microns. Preferably, the width and thickness of the channels is chosen so that the composite transducer element will contain from about 25 to 35 volume percent piezoelectric material with the remaining volume being substantially polymeric material.

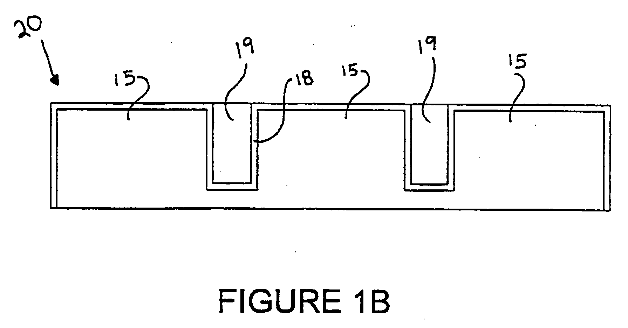

Following the formation of the first and second set of channels (i.e., dicing), a film 18 of a polymeric material is formed on the piezoelectric material and within the channels as shown in FIG. 1B. In general, film 18 is formed by filling the channels with a polymeric material before curing, excess polymer is removed by, for example, pressurized airing, and then followed by curing. As one skilled in the art can readily appreciate, film 18 can also be formed by other methods known to one skilled in the art, e.g., dip coating material 10 in a polymeric material, removing any excess polymer and then followed by curing. This will leave only a film of the polymer on the piezoelectric material. Suitable polymeric materials for forming the film include low viscosity Spurr epoxies, silicone and silane additives such as those available from Dow Coming, e.g., water-based acrylics, solvent-based acrylics, water-based polyesters, solvent-based polyesters, water-based alkyds, solvent-based alkyds, solvent-based amides, solvent-based nitrocellulose, water-based polyurethanes, solvent-based polyurethanes, solvent-based epoxies, water-based vinyls, solvent-based vinyls, solvent-based phenolics and the like with the Spurr epoxies being preferred.

Next, additional polymeric material 19 is deposited over the film to fill each of channels 12 and 14 to form a flexible composite 20 as depicted in FIG. 1B. Suitable polymeric materials for use herein include thermoplastics, thermosets, rubbers and mixtures thereof. Useful thermoplastics include high density polyethylenes, polymethyl methacrylates, polypropylenes, polybutylene terephthalates, polycarbonates, polyurethanes such as CA 118 and CA 128 available from Morton Chemical and estane polyester, and the like. Useful thermosets include epoxies such as Spurr epoxy and Stycast 80, and the like. Useful rubbers include silicone rubbers such as dispersion 236 available from Dow Corning and RTV-141 available from Rhone-Poulenc, Inc. and the like. Preferred polymeric materials for use herein include Stycast 1365-65 available from Emerson and Gumming, Deway and Almay Chemical division (Canton, MA) and Spurr epoxy available from Ernest F. Fullam Inc. (Schenectady, NY).

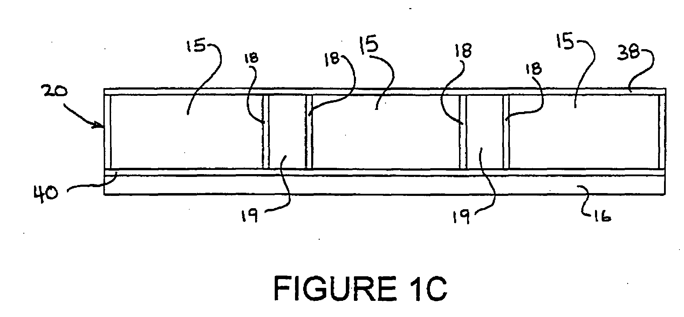

Following the depositing of polymeric material into each of channels 12 and 14, flexible composite 20 can be subjected to further processing to form the composite transducer element in accordance with the present invention. Further processing includes, for example, cutting composite 20 into slices and then polishing the surfaces thereof. Electrode surfaces 38 and 40 can then be applied to, and substantially coextensive with, opposite surfaces of the composite 20 (See FIG. 1C) by techniques known to one skilled in the art, e.g., sputtering, painting, etc. However, if the transducer elements are to be employed in an array to form a transducer array, the electrode surfaces may be applied at the time the array is formed as discussed below. Materials useful in forming electrode surfaces 38 and 40 include copper, silver, nickel, gold, alloys, mixtures thereof and the like.

Once electrode surfaces 38 and 40 have been applied to composite 20, a matching layer 16, which is discussed hereinbelow, can then be applied to, and substantially coextensive with, one of the electrode surfaces 38 and 40 (See Fig. 1C) to form the composite transducer element.

A second type of composite transducer element for use herein to form a large-area flexible transducer array can be formed from one or more fibers extruded from a composition containing a piezoelectric material. The extruded fibers can be knitted, woven or braided in 1, 2 or 3 dimensions to provide a structure that includes spaces which can be filled with polymeric material to form a composite. Fibers made of piezoelectric material suitable for use herein can be prepared using a viscous suspension spinning process and are available from Advanced Cerametrics Incorporated (Lambertville, NJ). Suitable piezoelectric materials for use in the fibers include PZT powders commercially available from Morgan Matroc, Inc., ceramic, lead zirconate titanate $Pb(Zr,Ti)O_3$, lead metaniobate $Pb(Nb_2O_6)$, modified lead titanate $PbTi_3$ such as $(Pb,Ca)TiO_3$ and $(Pb,Sm)TiO_3$, barium titanate $BaTiO_3$, PMN-PT$(1-x)Pb(Mg_{1/3}Nb_{2/3}O_3$-$xPbTiO_3$, PZN-PT/BT $Pb(Zn_{1/3}Nb_{2/3})O_3$-$xPbTiO_3$-$BaTiO_3$, $(1-x)Pb(Zn_{1/3}, Nb_{2/3})O_3$-$x(yPbTiO_3$-$(1-y)PbZrO_3)$ and the like. In particularly useful embodiments, the extruded fibers are knitted, woven, braided or non-woven to form a fiber sheet. Preferably, a simple woven fabric is used as the fiber sheet. The individual fibers in the fiber sheets can be coated with a film-forming polymer solution to improve the strength and wear resistance of the individual fibers prior to weaving, knitting and braiding. Suitable solutions of film-forming material are known to those in the textile art. By way of example, the solution can contain a mixture of a polyvinyl alcohol and polyvinyl acetate as a major component thereof and polyethylene glycol as a minor component thereof.

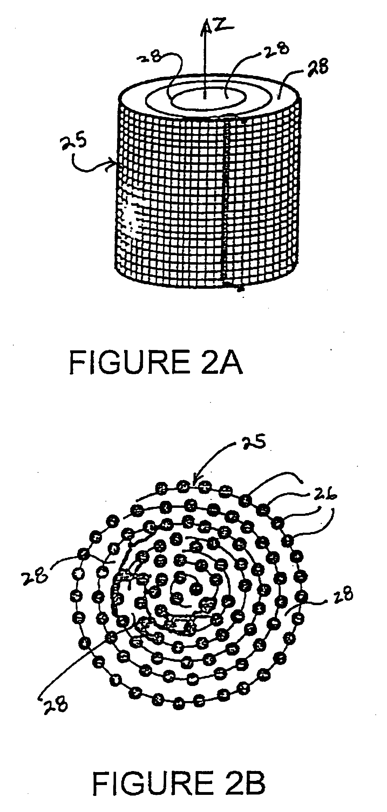

The fiber sheet 25 is rolled about a common axis (z) into a spiral configuration as depicted in FIG. 2A. When the preferred simple woven fabric is used as the fiber sheet, piezoelectric fibers 26 will be positioned parallel to the axis about which the fabric has been rolled as shown in FIG. 2B. The rolled fabric is then heat treated to achieve sintering using techniques known to those skilled in the art. Overlapping segments of the sintered spiral will define gaps 28. These gaps 28 are filled with a polymeric material to form a spiral composite. Suitable polymeric materials for use herein include thermoplastics such as high density polyethylenes, polymethyl methacrylates, polypropylenes, polybutylene terephthalates, polycarbonates, polyurethanes such as CA 118 and CA 128 available from Morton Chemical and estane polyester, and the like, thermosets such as epoxies, e.g., Spurr epoxy and Stycast 80, and the like; rubbers such as silicone rubbers such as dispersion 236 available from Dow Coming and RTV-141 available from Rhone-Poulenc, Inc. and the like and mixtures thereof. A preferred polymeric material for use herein is an epoxy such as Stycast 1365-65.

Before curing or hardening of the polymer, it may be necessary to subject the spiral composite to reduced pressure to remove any air babbles that may be trapped within the spiral. Once hardened or cured, the spiral transducer element can be cut into slices, polished, electroded and poled using techniques known to those skilled in the art. Following the further processing of the element, electrode surfaces can be applied to, and substantially coextensive with, opposite surfaces of the spiral element as discussed above. However, if the transducer elements are to be employed in an array to form a transducer array, the electrode surfaces may be applied at the time the array is formed as discussed below. Suitable materials for electrode surfaces include copper, silver, nickel, gold, alloy, mixtures thereof and the like. A matching layer, which is discussed hereinbelow, can then be applied to, and substantially coextensive with, one of the electroded surfaces to form the transducer element.

The composite transducer elements are tested for flexibility on an Instron device using the four point bend test described in ASTM method C 1161-90. Both the diced and spiral composites can withstand a 2mm bend without failure.

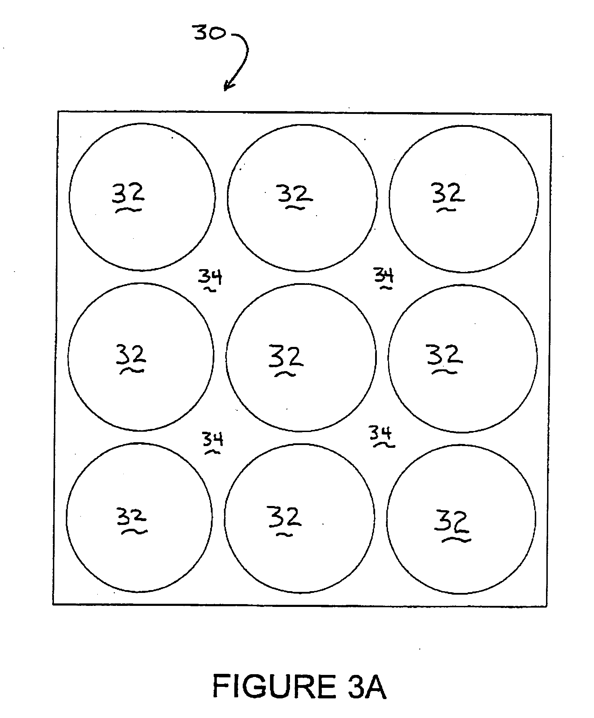

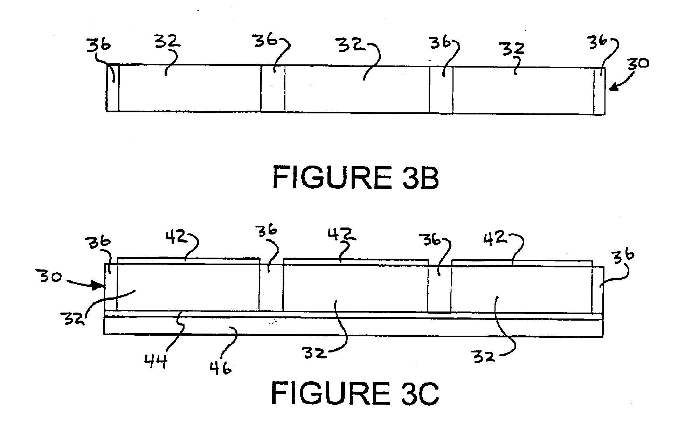

The flexible transducer array of the present invention can be obtained by forming an array of a plurality of either of the foregoing composite transducer elements. The array 30 can be formed, for example, by arranging spiral composite transducer elements 32 in adjacent relation thereby forming spaces 34 therebetween as generally depicted in FIG. 3A. It is to be understood that the transducer elements 32 can be of varying sizes when forming the array described herein. In general, the composite transducer elements can be placed close together or spaced further apart and the spacings need not be uniform or in perfect alignment. The composite transducer elements, however, are not initially secured to another. Thus, to form the array, the orientation of each composite transducer element is determined and placed flat in, for example, a mold, in the desired arrangement. Typically, the space between the composite transducer elements will range from about 0.5mm to about 10mm and preferably from about 1mm to about 3mm. A polymeric material 36 is then back-filled into the spaces formed between the arranged composite transducer elements to adhere the elements together and form a coherent array. See FIG. 3B. The dimensions of the array will typically be in the range of from about 0.5 inch to about 6 inches wide and from about 0.5 inch to about 12 inches long. The thickness of the array can effect the frequency of operation and will ordinarily range from about 0.05mm to about 10mm. It should, of course, be understood that the array can include elements with different frequencies of operation. These differences in frequency of operation can be achieved by employing composites of different thicknesses or having different levels of polymer loading. Suitable polymeric materials include those polymeric materials discussed above with the preferable polymeric material being Stycast 1365-65.

Once the array has been formed, electrode surfaces 42 and 44 are applied to opposite surfaces of the array as depicted in FIG. 3C. Typically, electrode surfaces 42 will be applied onto one surface of each transducer element in the array by techniques known to one skilled in the art, e.g., utilizing photolithographic techniques. In general, electrode surface 44 will be applied to, and substantially coextensive with, the other surface of the array. Electroding can be achieved employing techniques known to one skilled in the art, e.g., sputtering, painting. Materials useful in forming the electrode surfaces include copper, silver, nickel, gold, alloys, mixtures thereof and the like. Typically, electrode surfaces 42 will be positively charged and the other electrode surface 44 will serve as a ground.

Once electrode surfaces 42 and 44 have been applied, matching layer 46 can then be applied to, and substantially coextensive with, electrode surface 44, as shown in FIG. 3C. Techniques for applying the matching layer are within the purview of one skilled in the art. Generally, the thickness of the matching layer can be chosen to correspond to one-quarter of the wavelength in the matching layer at the operating frequency of the transducer array. The acoustic impedance of the matching layer will preferably range from about 2.0 to about 7.0 MRayls and more preferably from about 3.0 to about 4.0 MRayls.

Matching layer will ordinarily be formed from a polymeric material, and optionally, a filler. The polymeric material should have good compatibility with the components of the composite, biocompatibility and flexibility. Useful polymeric materials include thermoplastics such as high density polyethylenes, polymethyl methacrylates, polypropylenes, polybutylene terephthalates, polycarbonates, polyurethanes such as CA 118 and CA 128 available from Morton Chemical and estane polyester, and the like; thermosets such as epoxies such as Spurr epoxy and Stycast 80, and the like; and rubbers such as silicone rubbers such as dispersion 236 available from Dow Corning and RTV-141 available from Rhone-Poulenc, Inc. and the like. A preferred polymeric material for use herein is Stycast 1365-65. Because the acoustic impedance of many polymeric materials is less than the preferred range of 3.0 - 4.0 MRayls, it is necessary to increase the acoustic impedance of the polymer. Accordingly, one or more fillers can be incorporated therein. Suitable fillers include PZT, tungsten, alumina, silica glass, tungsten carbide, titanium, glass powder and the like with glass powder being preferred. The size of the filler particles should be in the range of about 0.1 to about 50 microns and preferably from about 0.5 to about 5 microns. The amount of filler employed will be that amount necessary to impart the desired acoustic impedance. Normally, from about 2 to about 50 percent filler by volume and preferably from about 5 to about 30 volume percent filler is employed.

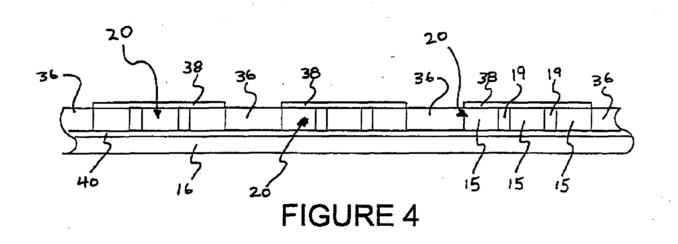

Alternatively, the large-area flexible transducer array can be obtained by forming an array of a plurality of the composite transducer elements formed from the aforementioned dicing and filling method. As depicted in FIG. 4, this transducer array will contain a plurality of the composite transducer elements 20 arranged in adjacent relation thereby forming spaces 36 which are back-filled with a polymeric material discussed above. Composite transducer element 20 will contain islands 15 of piezoelectric material having interconnecting channels which have a film formed thereon (not shown) and are then filled with a polymeric material 19. Next, electrode surfaces 38 are applied onto one surface of each composite transducer element 20 in the array employing techniques known in the art, e.g., photolithography techniques. Electrode surface 40 is then applied to, and substantially coextensive with, the other surface of the array. Then a matching layer 16 is applied to, and substantially coextensive with, electrode surface 40 as discussed above.

For convenience, a housing can be provided to at least partially surround the electroded flexible composite transducer element or array of such elements and any associated matching layer. Normally, the matching layer will not be covered by the housing to avoid undesired changes in the acoustic impedance of the outer most surface of the system. Alternatively, the matching layer can be integral with the housing. Any material can be used to form the housing. Preferably, biocompatible polymers are used. Where a flexible array of transducer elements is being produced, the housing material should be at least as flexible as the array. Polymeric materials are particularly suitable in this instance.

The transducer elements and transducer arrays of this invention are particularly useful in therapeutic applications. In general, the foregoing transducers will flex to conform to a part of the human anatomy, e.g., the hip, spine, etc., to facilitate the transfer of acoustic energy to promote healing of bone fractures and soft tissue wounds. Typically, the frequency of the acoustic energy will be in the range of from about 0.1 to about 10 MHz and preferably from about 0.5 to about 5 MHz. Features characterizing the frequency of the acoustic energy are disclosed in U.S. Patent No. 5,520,612, the contents of which are incorporated by reference herein. It is to be understood that multiple sections of the human anatomy can be treated with multiple transducer elements or transducer arrays at the same time. Thus, for example, in the case of an individual suffering from both a bone fracture in the hip and a soft tissue wound in the back, a transducer element or transducer array can be applied to the hip while a second transducer element or transducer array is being applied to the section of the back suffering from the wound. The transmit frequency and acoustic energy applied to each section can vary according to the foregoing ranges.

When applying the foregoing transducers to the section of the human anatomy in need of therapeutic assistance, it is advantageous to apply a coupling gel to the transducer prior to its employment on the part of the body. Additionally, when employing the foregoing transducer arrays, it may be desirable in certain cases to use selective energization of the transducer elements in the array such that certain elements will generate acoustic energy while other elements will not. This will permit the spatial, temporal and frequency control of the distribution of acoustic energy in the body. Techniques for altering the acoustic energy of the transducer elements are disclosed in U.S. Patent No. 5,520,612.

The following examples are illustrative of the present invention.

<u>EXAMPLE 1</u>

The following example is illustrative of the manufacturing of a piezoelectric composite using the dice and fill method described above and use of the composite in an array to form a transducer in accordance with the present invention.

The fabrication processing of the transducer consisted of three main parts: (I) ceramic sample preparation and characterization, (II) dicing an filing method, and (III) array fabrication and final characterization. All the samples were processed from Morgan Matroc, Inc. PZT-5H powders. The as received powders contained approximately 3 weight percent polyvinyl alcohol (PVA) binder. To remove the initial binder from the powders, an initial binder bum out (BBO) cycle was performed in a furnace at 550°C for 3 hours. After this BBO step, the powders were mixed with exactly 6 weight percent of PVA binder solution (PVA/water ratio was 20/80) in a mortar and pestle and then sieved through a 70 mesh sieve.

Several pellets of 1.75" in diameter were pressed uniaxially at 22 ksi. An average weight of 26 g of PZT+binder powder was used for each pellet. The average green density was computed to be 4.58 ±0.02 g/cc which is 58 percent of the theoretical density of PZT ($\rho_{th}$=7.86 g/cc). These pressed samples were subjected to a slow BBO cycle. An average of 1.15 weight percent loss was measured on the pellets after the BBO cycle. Sintering was carried out at 1285°C for 1 hour in a closed and sealed $Al_2O_3$ container. A mixture of $PbO+ZrO_2$ was used as a lead source to compensate for lead loss from the samples. In this step, the samples were placed on PZT-5H coarse powder (0.5-1.0 mm). An average of 0.3±0.06 weight percent lead loss was found in the samples after sintering. The mean sintering density, by measuring the mass and dimensions of the sample, was found to be 7.45±0.01 g/cc (approximately 94.7±0.2% of the theoretical value).

The sintered samples were then polished with a dicing saw. Next, a 1200 SiC grit paper was used to smooth the surfaces. Silver paint was applied on one of the faces of the pellets and poled by the corona poling method. In this technique, the unelectroded top surface of the sample is exposed to a shower of positive ions while the bottom surface is grounded to a metallic plate placed on a hot plate. A potential of 25 kV was applied to the corona needles, ionizing the air between the tips of the needles and the metallic grounded plate which was 45 millimeters away from the needle tips. Poling was done for 15 minutes with the temperature being 70°C during the poling process. Then, the silver was removed with acetone and both faces of the samples were gold sputtered. The samples were left to age for 24 hours at room temperature with the gold electrodes short circuited, before physical and electrical characterization.

Capacitance (Cp) and dissipation factor (tanδ) were measured at 1 kHz with a 1689M precision RLC Digibridge (GenRad Inc., Boston, MA). The relative dielectric constant K of a sample of thickness t was calculated by:

$$k = \frac{C_p t}{\varepsilon o A}$$

where $\varepsilon_o$ is the permitivity of free space (8.85X10$^{-12}$ F/m), and A is the electroded area. The piezoelectric charge coefficient $d_{33}$ of the composites was measured at 100 Hz using a Berlincourt Piezo $d_{33}$-Meter (CPDT-3300-Chanel Product, Inc., Cleveland, OH). Two flat probes were used to test the composite and two round probes were used for ceramic samples.

The thickness and planar coupling coefficients were calculated by measuring the resonance and antiresonance frequencies with an Impedance/Gain-Phase Analyzer (4194A Hewlett-Packard Inc., Palo Alto, CA) and applying the following equations:

$$k_p{}^2 = \frac{f_p - f_s}{f_s}\left[\frac{R_1{}^2 - (1 - \sigma^2)}{1 + \sigma^2}\right]$$

$$k_t{}^2 = \frac{\pi}{2}\frac{f_s}{f_p}\tan\left(\frac{\pi}{2}\frac{f_p - f_s}{f_p}\right)$$

where $k_t$ and $k_p$ are thickness and planar coupling coefficients, $f_p$-$f_s$ is the parallel and series resonance frequencies difference, and the value of $R_1$(≈ 2.05) is a function of Poisson's ratio for σ = 0.3. The average dielectric constant, dissipation factor, $d_{33}$, $k_t$ and $k_p$ coefficients of the pellet samples were 3021 ±36, 0.0187±0.0005, 608±5 pC/N, 56% and 73 % respectively.

The poled samples were then cut into a 1 x 1 in$^2$ plate and diced with a diamond-dicing saw into a modified 2-2 (or 2-(1-3)-2) connectivity pattern. The thickness of the ceramic wall (p), the spacing (e) between them and the connectivity were chosen to achieve the desired direction of flexibility and the volume fraction of the final element. The volume fraction of the piezoelectric diced ceramic ($V_c^f$) is related to p and e by:

$$V_c^f = \frac{p}{p+e} \times \frac{p'}{p'+e'}$$

The primed terms indicate wall and spacing dimensions, cut in different perpendicular directions. Low viscosity hard Spurr polymer was used to refill the diced ceramics elements. The polymer was then removed from the ceramic, leaving a thin spurr film behind. Curing of this film at 70 °C for 12 hours was performed.

The composites were then arranged in a 4x3 array and back-filled with epoxy as follows: twelve different one-inch squared PZT/polymer composite elements were arranged in a rectangular 4 x 3 army, with 1-mm separation between elements. The thickness was chosen to obtain a characteristic resonance frequency of 1MHz when the volume fraction of the PZT in the composite was equal to 30%. The single composites were placed in a plastic dish (with same polarity up or down) with the specified 4 x 3 arrangement. The positive sides of all elements were glued to the plastic dish to prevent any displacement during the curing stage since the polymer may expand during curing which can change the original layout of elements.

The arrays were back filled with Stycast 1365-65, de-aired, and cured. The cured array composites were polished to desired thickness corresponding to 1 MHz resonance frequency. Polishing was performed using a dicing saw (Kulicke & Soffa Industries Inc., Model 775 wafer saw, Horsham, PA). A 2 mm thick 340-mesh silicon carbide blade was used in this process. The blade swept the surface of array composite with 1-millimeter increment in x-y plane and 100 μm depth in z-direction. Since Stycast 1365-65 has higher Poisson's ratio than PZT ($\sigma \approx 0.3$), using this small increments of depth was necessary to obtain a uniformly polished surface after each sweep.

The negative sides of arrays were gold sputtered to form common electrodes. The gold sputtering was accomplished using a dc-sputter coating (Polaron Instruments, SE Coating Unit E5100, Doylestown, PA) at room temperature and 20 mA dc-current under -608 mmHg pressure (with argon gas flow) for 4 minutes. Re-poling is ordinarily not necessary in these diced composites arrays. Typical electromechanical properties of the PZT 2-(1-3)-2 array are set forth below in Table 1.

Table 1.

| Acoustic and electromechanical properties of 2-(1-3)-2 flexible array. | | | | | |
|---|---|---|---|---|---|
| K@1kHa | Tan$\delta$@1KHz | $d_{33}$ (pC/N) | v (m/s) | Z (Mrayls) | $k_t$ (%) |
| 423±20 | 0.0240±0.0006 | 400±16 | 3280±23 | 9 | 63±1 |

Single element and three-element array composites were tested for flexibility using the previously described four-point bend test and withstood a 2mm deflection without failure.

## EXAMPLE 2

This example illustrates the preparation of a spiral fiber composite and transducers employing the composites.

Continuous ceramic fibers were prepared using a viscous suspension spinning process. To generate ceramic fibers, 75 - 90 weight percent ceramic is added to viscose (cellulose) and the resulting spin mix produces green fibers with 50 to 76 volume percent ceramic-to-carrier loading. Filament diameters typically range from 25 to 70 μm in green stage and 10 to 30 μm after sintering. In this example, PZT-5H powder purchased from Morgan Matroc Inc. (Bedford, OH) was applied to viscose to form a spinnable suspension.

The green PZT fibers required sizing to increase their strength for handling. Thus, a coating the surface of continuous fibers were coated with a film-forming polymer solution to improve their strength and wear resistance for further processing. To prepare the sizing solution, PVA 107 and PVA 205 purchased from Air Products and Chemicals, Inc., (Allentown, PA) and polyethylene glycol (PEG) (Carbowax series 200 - 1450) purchased from Union Carbide Chemicals and Plastics Company Inc. (Danbury, CT) were used as major and minor sizing components, respectively. Ethylene glycol [EG] available from Fisher Scientific Co., Chemical Manufacturing Division (Fair Lawn, NJ) was added to the sizing solution as a plasticizer for both PVA and PEG in order to increase the flexibility of the sized fibers.

PZT fibers were then soaked and passed through a steel sizing die to compact and align the fiber bundle. The sizing die (Hoosier Ajax, Fort Wayne, IN) has 750 μm diameter to size 800 individual filaments. The product was a collimated multifilament tow (a bundle of hundreds of fibers), dried at room temperature. Then, green continuous sized tows containing 800 fibers were woven into plain fabrics. Fabrics were cut into 4 x 150 cm$^2$ strips, hand-rolled tightly, and wrapped with bundles of PZT fibers to support the structure and to keep shape during firing. The structure is hereafter called spiral. In this structure, PZT fibers were aligned in the z-direction while being reinforced with several PZT bundles lying parallel in x-y planes.

Next, the spiral structures were placed on top of platinum foils sitting in a large alumina crucible of 20 x 30 x 2.5cm³. The entire arrangement was placed into a furnace for binder burn out (BBO). In the first step of heat treatment, the spirals were heated at 550°C for 4 hours with a 1.5°C/min heating rate to ensure that all the organic had burned off. Then, a pre-firing was performed at 780°C for 1 hour at 1.5°C./min. to strengthen the spiral structure for handling. In the second step of heat treatment, a small crucible containing 20g lead zirconate was placed next to the spirals to provide a lead-rich atmosphere during sintering. This assembly was sealed with an inverted large crucible of the same size and sealed with Ca-aluminate cement and fired at 1285 °C for 40 minutes with a 3.5°C/min ramp.

After sintering, the spiral structures were placed in plastic dishes and filled with Stycast 1365-65. Then, the system was put in a vacuum chamber and de-aired at -760 mmHg for 30 minutes. After all the air bubbles were removed, dishes were placed in an oven with preset temperature of 72° for 12 hours until curing was complete. The chamber was slowly cooled to room temperature at 0.1°C/min cooling rate to prevent warping and cracking of samples.

The cured spiral composites were sliced into 2.5mm thick samples and then polished to a thickness of 2 mm with 240, 400, and 500 grit SiC sand papers. One-inch diameter discs were cut out of each sample and dried at 72°C for 4 hours.

Physical properties of the spiral samples, including density and ceramic volume fraction in composites, were calculated from the following equations:

$$\rho_{composite} = M/V$$

$$V_{PZT} = [(\rho_{composite} - \rho_{polymer})/(\rho_{PZT} - \rho_{polymer})] * 100$$

where, $M$, $V$, and $\rho_{composite}$ are the weight, bulk volume, and bulk density of the composite, respectively. $V_{PZT}$ is the volume fraction of PZT ceramic of the composite, $\rho_{polymer}$ is the density of Stycast 1365-65 (approximately 1.1 g/cm³), and $\rho_{PZT}$ is the theoretical density of PZT ceramic (7.9 g/cm³).

The composites were then electroded with silver paint on one face only and poled via the corona method. In this technique the unelectroded top surface of the sample is exposed to a shower of positive ions while the bottom surface is grounded to a metallic plate placed on a hot plate. A potential of 26 kV is applied to the corona needles, ionizing the air between the tips of the needles and the metallic grounded plate, which was 45 millimeters away from the needle tips. Poling was done for 20 minutes and the temperature was dropped from 60 to 45 °C during the poling process.

After poling, the other sides of spiral composites were electroded with air-dried silver paint and dried at room temperature. Samples were aged for 24 hours before electromechanical evaluation.

Three twelve-element 4 x 3 array transducers were fabricated using spiral composites. The spiral composites were placed in a plastic dish (with same polarity up or down) in the specified 4 x 3 arrangement with each element being 1mm apart. The positive sides of all elements were glued to the plastic dish to prevent any displacement during polymer curing.

The arrays were back-filled with Stycast 1365-65, de-aired, and cured as discussed before. The cured array composites were polished to the desired thickness corresponding to 1 MHz resonance frequency. Polishing was performed using a dicing saw (Kulicke & Soffa Industries Inc., Model 775 wafer saw, Horsham, PA). A 2 mm thick 340-mesh silicon carbide blade was used in this process. The blade swept the surface of array composite with a 1-millimeter increment in the x-y plane and 100 μm depth in the z-direction.

The negative sides of arrays were the gold sputtered to form common electrodes. The gold sputtering was accomplished using a dc-sputter coating (Polaron Instruments, SE Coating Unit E5100, Doylestown, PA) at room temperature and 20mA de-current under -608 mmHG pressure (with argon gas flow) for 4 minutes.

The arrays were re-poled by the same technique previously discussed. Re-poling was necessary due to thermally and mechanically depolarizations, caused by the processing steps.

Individual elements on the top surface of arrays were gold sputtered. In this step, proper masks were prepared to cover the spacing between transducer elements. The masks allowed only the top surfaces of single transducers to be exposed to the gold target. The sputtering conditions were similar to the previous sputtering step. The electromechanical properties of the spiral transducer elements are set forth below in Table 2.

Table 2.

| Physical and Electromechanical Properties of Spiral Transducer Elements | | | | | | | | |
|---|---|---|---|---|---|---|---|---|
| Sample No. | $\rho$(g/cc) | $V_{PZT}$(%) | Tan$\delta$ | K | $d_{33}$(pC/N) | $k_t$(%) | $V_{sound}$ (m/s) | $f_s$(MHz) |
| 1 | 3.4 | 34 | 0.0333 | 370 | 368 | 63 | 2853 | 1.18875 |
| 2 | 3.3 | 32 | 0.0428 | 362 | 374 | 65 | 2622 | 1.09250 |

Table 2.   (continued)

| Physical and Electromechanical Properties of Spiral Transducer Elements | | | | | | | | |
|---|---|---|---|---|---|---|---|---|
| Sample No. | $\rho$(g/cc) | $V_{PZT}$(%) | Tan$\delta$ | K | $d_{33}$(pC/N) | $k_t$(%) | $V_{sound}$ (m/s) | $f_s$(MHz) |
| 3 | 3.3 | 32 | 0.0381 | 348 | 423 | 66 | 2607 | 1.08625 |
| 4 | 3.3 | 32 | 0.0441 | 277 | 418 | 65 | 2642 | 1.10100 |
| 5 | 3.2 | 31 | 0.0336 | 334 | 365 | 63 | 2844 | 1.18500 |
| 6 | 3.2 | 31 | 0.0356 | 313 | 349 | 62 | 2667 | 1.11150 |
| 7 | 3.3 | 32 | 0.0362 | 332 | 405 | 66 | 2548 | 1.06200 |
| 8 | 3.3 | 32 | 0.0446 | 324 | 440 | 63 | 2693 | 1.12200 |
| 9 | 3.3 | 32 | 0.0408 | 349 | 474 | 65 | 2542 | 1.05900 |
| 10 | 3.3 | 32 | 0.0402 | 323 | 425 | 65 | 2570 | 1.07100 |
| 11 | 3.4 | 34 | 0.0439 | 354 | 346 | 66 | 2650 | 1.10400 |
| 12 | 3.3 | 32 | 0.0411 | 352 | 410 | 64 | 2772 | 1.15500 |
| Average | 3.3 | 32 | 0.0395 | 337 | 400 | 65 | 2668 | 1.11150 |

Single element and three-element array composites were tested using the previously described four point bend test and were found to flex at least 2mm without failure.

Although the present invention has been described in preferred forms with a certain degree of particularity, many changes and variations are possible therein and will be apparent to those skilled in the art after reading the foregoing description. For example, while the foregoing description relates to medical applications, one skilled in the art would appreciate that the flexible composite transducer elements and large-area arrays of such transducer elements would also be used for medical diagnostic ultrasound applications and nondestructive testing such as, for example, nonmedical applications. As another example, instead of linear intersecting channels, spaces for receiving polymer can be provided in other configurations (e.g., pores, voids, arcuate channels, etc.) to achieve the desired volume of polymer loaning. In such embodiments, a film of a first polymeric material would be applied in the spaces in the piezoelectric material and then the spaces would be filed with a second polymeric material in accordance with the previously described embodiments. It is therefore to be understood that the present invention may be practiced otherwise than as specifically described herein without departing from the spirit and scope thereof.

## Claims

1.  A piezoelectric composite transducer element comprising:

    a fiber sheet formed from fibers extruded from a composition containing a piezoelectric material, the fiber sheet being rolled about a common axis to define a generally spiral configuration, overlapping segments of the spiral defining a gap therebetween, the gap being filled with a first polymeric material.

2.  The transducer element of Claim 1 wherein the fiber sheet is a fabric woven from one or more fibers containing a piezoelectric material.

3.  The transducer element of Claim 1 wherein the piezoelectric material is selected from the group consisting of PZT powder, ceramic, lead zirconate titanate $Pb(Zr,Ti)O_3$, lead metaniobate $Pb(Nb_2O_6)$, modified lead titanate $PbTi_3$, $(Pb,Ca)TiO_3$, $(Pb,Sm)TiO_3$, barium titanate $BaTiO_3$, PMN-PT$(1-x)Pb(Mg_{1/3}Nb_{2/3})O_3$-$xPbTiO_3$, PZN-PT/BT $Pb(Zn_{1/3}Nb_{2/3})O_3$-$xPbTiO_3$-$BaTiO_3$, $(1-x)$ $Pb(Zn_{1/3}, Nb_{2/3})O_3$-$x(yPbTiO_3$-$(1-y)PbZrO_3)$ and mixtures thereof.

4.  The transducer element of Claim 1 wherein the first polymeric material is selected from the group consisting of thermoplastics, thermosets, rubbers and mixtures thereof.

5.  The transducer element of Claim 1 wherein the first polymeric material is an epoxy.

6. The transducer element of Claim 1 wherein the extruded fibers possess a coating formed thereon.

7. The transducer element of Claim 1 wherein the coating comprises a film-forming polymer.

8. The transducer element of Claim 1 wherein the extruded fibers are sized in a polymer solution containing a polymer selected from the group consisting of polyvinyl alcohol, polyvinyl acetate and mixtures thereof as a major component thereof and polyethylene glycol as a minor component thereof.

9. The transducer element of Claim 1 further comprising an electrode surface applied to, and substantially coextensive with, opposite surfaces of the spiral configuration and a matching layer applied to, and substantially coextensive with, one of the electrode surfaces.

10. The transducer element of Claim 9 wherein the matching layer comprises a second polymeric material and optionally a filler.

11. The transducer element of Claim 10 wherein the second polymeric material is selected from the group consisting of thermoplastics, thermosets, rubbers and mixtures thereof.

12. The transducer element of Claim 10 wherein the second polymeric material is an epoxy.

13. The transducer element of Claim 10 wherein the matching layer includes a filler selected from the group consisting of PZT, tungsten, alumina, silica glass, tungsten carbide and titanium.

14. The transducer element of Claim 10 wherein the matching layer includes glass powder as a filler.

15. The transducer element of Claim 1 wherein the matching layer has an acoustic impedance of from about 2.0 to about 7.0 MRayls.

16. The transducer element of Claim 15 wherein the acoustic impedance of the matching layer is from about 3.0 to about 4.0 MRayls.

17. The transducer element of Claim 1 having a flexibility of at least 2 mm.

18. The transducer element of Claim 1 wherein the fiber sheet is knitted, braided or woven from extruded fibers containing a piezoelectric material.

19. The transducer element of Claim 1 wherein the piezoelectric matenal is PZT, the first polymeric material is an epoxy and the transducer element has a flexibility of at least about 2mm.

20. A flexible piezoelectric transducer array comprising:

an array comprising a plurality of composite transducer elements, each transducer element having a fiber sheet formed from fibers extruded from a composition containing a piezoelectric material and rolled about a common axis to define a generally spiral configuration, overlapping segments of the spiral defining a gap therebetween, the gap being filled with a first polymeric material, the plurality of composites arranged in adjacent relation to define spaces therebetween, the spaces being filled with a second polymeric material; one electrode surface applied to each transducer element on one surface of the array and a second electrode surface applied to, and substantially coextensive with, the other surface of the array; and, a matching layer applied to, and substantially coextensive with, the second electrode surface.

21. The transducer array of Claim 20 wherein the piezoelectric material is selected from the group consisting of PZT powder, ceramic, lead zirconate titanate $Pb(Zr,Ti)O_3$, lead metaniobate $Pb(Nb_2O_6)$, modified lead titanate $PbTi_3$, $(Pb,Ca)TiO_3$, $(Pb,Sm)TiO_3$, barium titanate $BaTiO_3$, PMN-PT$(1-x)Pb(Mg_{1/3}Nb_{2/3})O_3$-$xPbTiO_3$, PZN-PT/BT Pb $(Zn_{1/3}Nb_{2/3})O_3$-$xPbTiO_3$-$BaTiO_3$, $(1-x)$ $Pb(Zn_{1/3},\ Nb_{2/3})O_3$-$x(yPbTiO_3$-$(1-y)PbZrO_3)$ and mixtures thereof.

22. The transducer array of Claim 20 wherein the first polymeric material of the composite is selected from the grow consisting of thermoplastics, thermosets, rubbers and mixtures thereof.

**23.** The transducer array of Claim 20 wherein the first polymeric material is an epoxy.

**24.** The transducer array of Claim 20 wherein the extruded fibers possess a coating formed thereon.

**25.** The transducer array of Claim 24 wherein the coating comprises a film-forming polymer.

**26.** The transducer array of Claim 25 wherein the extruded fibers are sized in a polymer solution containing a polymer selected from the group consisting of polyvinyl alcohol, polyvinyl acetate and mixtures thereof as a major component thereof and polyethylene glycol as a minor component thereof.

**27.** The transducer array of Claim 20 wherein the matching layer comprises a third polymeric material and optionally a filler.

**28.** The transducer array of Claim 27 wherein the third polymeric material is selected from the group consisting of thermoplastics, thermosets, rubbers and mixtures thereof.

**29.** The transducer array of Claim 27 wherein the third polymeric material is an epoxy.

**30.** The transducer array of Claim 27 wherein the matching layer includes a filler selected from the group consisting of PZT, tungsten, alumina, silica glass, tungsten carbide and titanium.

**31.** The transducer array of claim 27 wherein the matching layer includes glass powder as a filler.

**32.** The transducer array of Claim 20 wherein the matching layer has an acoustic impedance of from about 2.0 to about 7.0 MRayls.

**33.** The transducer array of Claim 32 wherein the acoustic impedance of the matching layer is from about 3.0 to about 4.0 MRayls.

**34.** The transducer array of Claim 20 having a flexibility of at least about 2.0 mm.

**35.** The transducer array of Claim 20 wherein the fiber sheet is knitted, braided or woven from extruded fibers containing a piezoelectric material.

**36.** The transducer array of Claim 20 wherein the piezoelectric material is PZT, the first polymeric material is an epoxy and the transducer array has a flexibility of at least 2mm.

**37.** A method for manufacturing a flexible piezoelectric transducer array comprisirg:

a) forming a composite transducer element by

i) rolling a fiber sheet formed from fibers extruded from a composition containing a piezoelectric material into a spiral configuration, overlapping segments of the spiral defining a gap therebetween; and
ii) filling the gap with a first polymeric material;

b) forming an array by positioning a plurality of the composite transducer elements in adjacent relation to define spaces therebetween;
c) filling the spaces between the plurality of positioned composites with a second polymeric material;
d) applying one electrode surface to each transducer element on one surface of the array and a second electrode surface applied to, and substantially coextensive with, the other surface of the array; and,
e) applying a matching layer applied to, and substantially coextensive with, the second electrode surface.

**38.** The method of Claim 37 wherein the piezoelectric material is selected from the group consisting of PZT powder, ceramic, lead zirconate titanate $Pb(Zr,Ti)O_3$, lead metaniobate $Pb(Nb_2O_6)$, modified lead titanate $PbTi_3$, $(Pb,Ca)$ $TiO_3$, $(Pb,Sm)TiO_3$, barium titanate $BaTiO_3$, PMN-PT $(1-x)Pb(Mg_{1/3}Nb_{2/3})O_3$-$xPbTiO_3$, PZN-PT/BT $Pb(Zn_{1/3}Nb_{2/3})$ $O_3$-$xPbTiO_3$-$BaTiO_3$, $(1-x)$ $Pb(Zn_{1/3}, Nb_{2/3})O_3$-$x(yPbTiO_3$-$(1-y)PbZrO_3)$ and mixtutes thereof.

**39.** The method of Claim 37 further comprising the step of forming the fiber sheet by knitting, braiding or weaving the extruded fibers.

40. The method of Claim 39 further comprising applying a coating to the extruded fibers prior to forming the fiber sheet.

41. The method of Claim 40 wherein the coating comprises a film-forming polymer.

42. The method of Claim 39 wherein the extruded fibers are sized in a polymer solution containing a polymer selected from the group consisting of polyvinyl alcohol, polyvinyl acetate and mixtures thereof as a major component thereof and polyethylene glycol as a minor component thereof prior to forming the fiber sheet.

43. The method of Claim 37 wherein the first polymeric material is selected from the group consisting of thermoplastics, thermosets, rubbers and mixtures thereof.

44. The method of Claim 37 wherein the first polymeric material is an epoxy.

45. The method of Claim 37 wherein the second polymeric material is selected from the group consisting of thermoplastics, thermosets, rubbers and mixtures thereof.

46. The method of Claim 37 wherein the second polymeric material is an epoxy.

47. The method of Claim 37 wherein the matching layer comprises a third polymeric material and optionally a filler.

48. The method of Claim 47 wherein the third polymeric material is selected from the group consisting of thermoplastics, thermosets, rubbers and mixtures thereof.

49. The method of Claim 47 wherein the third polymeric material is an epoxy.

50. The method of Claim 47 wherein the matching layer includes a filler selected from the group consisting of PZT, tungsten, alumina, silica glass, tungsten carbide and titanium.

51. The method of Claim 47 wherein the matching layer includes glass powder as a filler.

52. The method of Claim 37 wherein the matching layer has an acoustic impedance of from about 2.0 to about 7.0 MRayls.

53. The method of Claim 52 wherein the acoustic impedance of the matching layer is from about 3.0 to about 4.0 MRayls.

54. The method of Claim 37 wherein the transducer has a flexibility of at least about 2.0 mm.

55. The method of Claim 37 wherein the piezoelectric material is PZT, the first polymeric material is an epoxy and the transducer array has a flexibility of at least 2mm.

56. A method of accelerating the healing of soft tissue wounds and bone fractures comprising:

positioning at least one flexible composite transducer element adjacent to a body at the site of a soft tissue wound or bone fracture,
the flexible composite transducer element including a fiber sheet formed from fibers extruded from a composition containing a piezoelectric material, the fiber sheet being rolled about a common axis to define a generally spiral configuration, overlapping segments of the spiral defining a gap therebetween, the gap being filled with a first polymeric material; and
causing the transducer to generate ultrasonic pulses.

57. A method as in Claim 56, wherein an array of said flexible composite transducer elements are positioned adjacent to the body.

58. A method as in Claim 56 wherein at least one flexible composite transducer element is positioned adjacent to a body at a plurality of sites of soft tissue wounds or bone fractures.

**59.** A method as in Claim 58 wherein arrays of said flexible composite transducer elements positioned adjacent to a body at a plurality of sites.

**60.** A flexible composite transducer element comprising

one or more fibers extruded from a composition containing a piezoelectric material, the fibers being arranged to define a structure including openings;

a first polymeric material substantially filling the openings, the transducer element having a flexibility of at least 2mm.

# FIGURE 1A

FIGURE 1B

FIGURE 1C

FIGURE 2A

FIGURE 2B

FIGURE 3A

FIGURE 3B

FIGURE 3C

FIGURE 4